# EUROPEAN PATENT SPECIFICATION

(11) **EP 2 782 899 B1**
(45) Date of publication and mention of the grant of the patent: **01.01.2020**
(21) Application number: 12801483.4
(22) Date of filing: 22.11.2012
(51) Int. Cl.: C07C 213/10, C07C 215/40, C07C 239/10, C01B 17/66

(54) **STABILIZED CHOLINE SOLUTIONS AND METHODS FOR PREPARING THE SAME**
STABILISIERTE CHOLINLÖSUNGEN UND VERFAHREN ZU IHRER HERSTELLUNG
SOLUTIONS DE CHOLINE STABILISÉES ET LEURS PROCÉDÉS DE PRÉPARATION

(30) Priority: 22.11.2011 WO PCT/US2011/061826
(43) Date of publication of application: 01.10.2014
(73) Proprietor: Taminco, 9000 Gent (BE)
(72) Inventor: MOONEN, Kristof, 9220 Hamme (BE); GERNON, Michael, David, Pace, Florida 32571 (US)
(74) Representative: Gevers Patents
(86) International application number: PCT/EP2012/073337
(87) International publication number: WO 2013/076190

(56) References cited:
- EP-A2- 1 561 747
- WO-A1-2006/005692
- FR-A- 1 171 967
- JP-A- 59 134 752
- JP-A- S59 134 752
- US-A- 4 425 202
- US-A- 4 464 461
- US-A- 4 686 002
- US-A- 5 209 858

## Description

### FIELD OF THE INVENTION

The invention relates to stabilized choline hydroxide solutions and the methods for preparing such stabilized solutions.

### BACKGROUND OF THE INVENTION

Choline generally refers to the various quaternary ammonium compounds containing the N,N,N-trimethyl ethanol ammonium cation. One specific form is the combination with the hydroxide anion, which is known as choline hydroxide.

Choline hydroxide is a strong base which has applications in the production of other choline salts, for example, by neutralization with an appropriate acid or in applications where a strong base containing very low levels of inorganic ions is needed. For instance, a choline base, such as choline hydroxide, is important in applications such as in manufacturing electronics.

Choline hydroxide is by itself a rather unstable molecule, however, and the degradation of choline base occurs fairly readily with the formation of usually undesired byproducts (e.g. trimethylamine and enal polymers). For instance, the degradation of choline base may occur by a process often referred to as Hofmann elimination. In Hofmann elimination, a basic molecule abstracts a proton from a carbon atom which is located in a beta position relative to a carbon atom bearing a good or suitable leaving group. The residual negative charge which is then left at the beta position after proton abstraction forms a double bond with the alpha carbon bearing the leaving group, and, in the process of forming the double bond, ejects the leaving group. In the case of choline hydroxide, the hydroxide counter anion abstracts a proton from the hydroxymethylene group carbon atom followed by ejection of the adjacent trimethylamine group. Thus, the products of Hofmann elimination from choline base are trialkyl amine, most commonly trimethylamine, and acetaldehyde.

Acetaldehyde may in its turn take part in subsequent sequential aldol condensations, which may readily be catalyzed by the presence of ample strong base, to yield conjugated polyunsaturated enal polymers with high colour. In addition, the trimethylamine byproduct is highly volatile and has a strong odour.

Therefore, the formation of undesired byproducts, such as enal polymers and trimethylamine, for example, may result in negative consequences, such as the rapid development of heavy or dark colour, and also to the formation of precipitates from the choline base solution, volatility, a strong smell, etc. Thus, degradation reactions usually deteriorate the quality of the choline hydroxide solutions and typically make them useless for most of its applications.

Choline base stabilizers generally are designed to deal with the acetaldehyde that is liberated during the Hofmann elimination reaction. The fast "scavenging" (e.g. reduction) of acetaldehyde removes the raw material necessary for the sequential aldol condensations which produce coloured polymers.

Thus, chemicals that react readily, reduce and/or disruptively copolymerize with acetaldehyde, such as formaldehyde, hydroxylamine, and semicarbazide, have been found to be good stabilizers for choline hydroxide. Formaldehyde, hydroxylamine, and semicarbazide have limited utility however, due to potential toxicity issues and/or concerns.

Additionally, sulphites have been used as a means for stabilizing choline base and related quaternary hydroxyethyl ammonium hydroxide compounds, for example. Sulphites are generally required at high concentration however, in order to be effective.

Additionally, borohydrides and aluminohydrides may reduce acetaldehyde to the corresponding alcohol (i.e., ethanol) and may reduce conjugated enal polymers to the corresponding alcohol with some reduction of the conjugated polyene function also occurring. There is a safety risk however, associated with the use of the hydride reducing agents, such as borohydride, even in an aqueous solution, because hydrogen gas may evolve, for example, upon neutralization of aqueous choline hydroxide solutions in final applications wherein another choline salt is being produced. Hydrogen is notorious for having the broadest explosive range in mixtures with air, and hence represents a significant safety hazard which is important to avoid.

Japanse patent application JP S 59-134752 discloses the stabilization in view of discoloration of quaternary ammonium hydroxide compositions such as trimethyl monoethanolamine hydroxide (choline hydroxide) by addition of a N,N-dialkylhydroxylamine, more particularly N,N-diethylhydroxylamine.

Thus, there remains a need for an effective stabilizer for choline hydroxide solutions to minimize or eliminate degradation reactions and the formation of undesired byproducts, without the currently identified concerns about possible toxic effects or the known safety risks, and where the stabilizer may be effective even when added in small amounts.

### SUMMARY OF THE INVENTION

Aspects of the present invention include methods for the stabilization of aqueous choline hydroxide solutions and stabilized choline hydroxide solutions, which includes the selection of an effective stabilizer or stabilizers in small amounts (e.g. less than about 5000 ppm by weight of the total solution) which reduce or eliminate the degradation and/or decomposition reactions and the formation of undesired byproducts without causing the concerns about possible toxic effects or about safety risks.

Additionally, it was discovered that although a significant amount of the degradation of choline hydroxide may occur via the process designated as Hofmann elimination, it may probably not be the only degradation process occurring. The degradation of choline base may also be accelerated by other agents, such as oxygen, which are not generally known to be important in the Hofmann elimination. Amongst the other degradation processes which are likely to be occurring, oxidation is believed to be particularly significant. Additionally, the concentration of choline hydroxide in the solutions may by itself influence the amount, level or rate of degradation (e.g., colour formation) where a higher concentration of choline hydroxide may for instance lead to more rapid and darker development of colour.

The present invention provides for stabilized aqueous choline hydroxide solutions (even at higher concentrations of choline hydroxide), which have a low and stable colour (e.g. remaining substantially clear) after synthesis and for a period of at least one to several months in storage at room temperature.

The choline hydroxide solution may be stabilized using a dithionite salt (e.g. sodium dithionite) as a sole stabilizer or, alternatively, in combination with small amounts of dialkyl hydroxylamine or other stabilizers, as disclosed in copending patent application PCT/US2011/061826.

In one embodiment, the invention provides for a method wherein the choline hydroxide solution is stabilized using a dialkyl hydroxylamine (e.g. N,N-diethyl hydroxylamine) as the sole stabilizer or, alternatively, in combination with small amounts of a dithionite salt or other stabilizers.

The stabilizers used herein are effective at low levels (e.g. 0.1% or lower) and may reduce the degradation reactions associated with one or both of the Hofmann elimination and the other degradation processes, such as oxidation, autoxidation, and the like.

According to one aspect, the present invention provides a method for the stabilization of an aqueous choline hydroxide solution including the adding of a second stabilizer comprising a dialkylhydroxylamine to the aqueous choline hydroxide solution after the aqueous choline hydroxide solution has been formed. A first stabilizer comprising a dialkyl hydroxylamine is added to an aqueous solution containing reactants that will eventually produce the aqueous choline hydroxide solution.

According to the invention, the method for the stabilization of the aqueous choline hydroxide solution includes adding the first stabilizer comprising a dialkyl hydroxylamine to the aqueous solution containing reactants that eventually will produce the aqueous choline hydroxide solution; and after the aqueous choline hydroxide solution has been formed, adding the second stabilizer of the dialkyl hydroxylamine to the aqueous choline hydroxide solution. The second stabiliser may further comprise a dithionite salt.

According to another aspect, the invention provides a stabilized choline hydroxide solution comprising choline hydroxide, water, and a stabilizer comprising a dialkyl hydroxylamine.

In another embodiment, the stabilizer in the stabilized choline hydroxide solution according to the present invention further comprises a dithionite salt.

The applicants have surprisingly found that the choline hydroxide solution according to the present invention, as well as the solution as produced by the method according to the present invention, remain stable for a long period even at the unconventionally low concentrations of the stabilizer or of the combination of stabilizers.

### DETAILED DESCRIPTION OF THE INVENTION

Aspects of the present invention include methods of stabilizing choline base solutions and the resulting stabilized choline solutions. As used herein, the terms "stabilizing" and "stabilized" are intended to encompass a choline hydroxide solution that undergoes minimal or no degradation reactions that would otherwise deteriorate the quality of the choline hydroxide solutions. In other words, there is reduced or no development of heavy, usually dark, colour, formation of precipitates, volatility, a strong smell, etc. Instead, the stabilized choline solution may maintain a clear or slightly off-colour (e.g. APHA of less than 500) appearance for an extended period of time (e.g. at least one week, at least one month, at least three months, etc.) at room temperature (e.g. about 20-25°C) under further under standard conditions. Additionally, the stabilized choline solution may also maintain a clear or slightly off-colour (e.g. APHA of less than 500) appearance even if subjected to elevated temperatures (e.g. up to 60°C).

According to one embodiment of the invention, a stabilized choline hydroxide solution comprises choline hydroxide, water, and a stabilizer comprising a dialkylhydroxylamine.

As used herein and in the claims, the terms "comprising" and "including" are inclusive or open-ended and do not exclude additional unrecited elements, compositional components, or method steps. Accordingly, the terms "comprising" and "including" encompass the more restrictive terms "consisting essentially of" and "consisting of." Unless specified otherwise, all values provided herein include up to and including the endpoints given, and the values of the constituents or components of the compositions are expressed in weight percent or % by weight of each ingredient in the composition. Additionally, each compound used herein may be discussed interchangeably with respect to its chemical formula, chemical name, abbreviation, etc.

### Choline Hydroxide Solutions

The stabilized choline hydroxide solution is a solution, which includes a choline base, such as choline hydroxide. Choline, also known as choline base, is a colourless liquid and a strong organic base. Chemically, it is within the context of the present invention limited to the narrow meaning of trimethyl-(2-hydroxyethyl)-ammonium hydroxide and may be represented by the formula:

[(CH₃)₃ N - CH₂ - CH₂ - OH]⁺ OH⁻

Choline hydroxide, also known as (2-hydroxyethyl) trimethyl-ammonium hydroxide, is an organic base suitable for many uses. For example, aqueous solutions of choline base are useful in connection with electronic applications, such as positive photoresist developing agents, stripping photoresists, anisotropic etching agents, and washing agents for silicon wafers.

The stabilized choline hydroxide solution may be in any suitable form. In one embodiment, the choline hydroxide is an aqueous choline solution, which includes a choline base and water. The preferred aqueous medium is water although other aqueous solvents including polar aprotic solvents may also be suitable. The water may be of any suitable type, e.g., distilled, deionised, treated, etc. Preferably, the water is in pure form with little to no impurities. The type and amount of aqueous medium is not especially limited, but may be employed in amounts sufficient to achieve a homogenous solution.

The solution may comprise any suitable concentration of choline hydroxide. The concentration of choline hydroxide in the solution may be high (for example, on the order of about 30 to about 60 weight %, about 40 to about 50 weight % choline hydroxide, or about 45 weight % choline hydroxide) based on the total amount of the aqueous choline hydroxide solution.

The aqueous choline hydroxide solution may be prepared using any suitable reactants and reaction mechanisms known by one of ordinary skill in the art. For example, the choline hydroxide solution may be prepared by the reaction of trimethylamine (TMA) with ethylene oxide (EO) and one equivalent of water in an aqueous solvent. The solvent is preferably pure water. A substantial excess of water may be necessary to dissipate the heat generated by the strongly exothermic reaction. Other reactants, solvents, catalysts, etc. may also be added with the primary reactants as will be appreciated by one of ordinary skill in the art. Additionally, any pre-treatments, such as pre-treating the water with trimethylamine in the case where the stabilizer hydrolyzes at a neutral or acid pH, may also be performed as needed.

### Dialkyl hydroxylamine Stabilizer(s)

According to one embodiment of the invention, the stabilizer for the stabilized choline hydroxide solution comprises at least one dialkyl hydroxylamine, such as N,N-dialkyl hydroxylamine. The dialkyl hydroxylamine may be of the following formula:

X₁X₂NOH

where X1 and X2 independently represent an alkyl group. The alkyl groups may include any linear or branched chain alkyl groups comprising one or more carbon atoms. For example, the alkyl groups may include 1 to 10 carbon atoms (e.g., methyl, ethyl, propyl, etc.). Suitable dialkyl hydroxylamines may include, but are not limited to, diethyl hydroxylamine, di-isopropyl hydroxylamine, and the like. In an exemplary embodiment, the dialkyl hydroxylamine comprises N,N-diethyl hydroxylamine (DEHA).

The dialkyl hydroxylamine may be obtained and added to the choline solution in any suitable form (e.g., aqueous). For example, diethyl hydroxylamine may be in anhydrous or aqueous (dilute) form and may be manufactured by the reaction of a triethylamine and peroxide, followed by Cope Elimination, purification and distillation.

In one embodiment of the invention, the dialkyl hydroxylamine stabilizer is the sole stabilizer used to stabilize the choline solution. As noted above, "sole" stabilizer is intended to mean that only that ingredient is intended to stabilize the choline solution by minimizing or eliminating degradation reactions (e.g., Hofmann elimination, oxidation, decomposition, etc.) that lead to colour change. Thus, in this embodiment, the dialkyl hydroxylamine acts as the only stabilizer or is effective as the sole stabilizer in the aqueous choline hydroxide solutions.

In another embodiment of the invention, the stabilizer comprises dialkyl hydroxylamine, such as N,N-diethyl hydroxylamine, as the primary stabilizer (e.g. at least 50 % by weight of the stabilizer is at least one dialkylhydroxylamine), with lesser amounts of additional stabilizers. The dialkyl hydroxylamine acts as the primary stabilizer in preventing colour formation and preserving the overall quality of the product. The dialkyl hydroxylamine acts as the primary stabilizer by including at least a ratio of 1:1 or at least 2:1 dialkyl hydroxylamine to additional stabilizer. In other words, the ratio of dialkyl hydroxylamine to additional stabilizer may range from about 1:1 to 10:1 or about 2:1 to 4:1 dialkyl hydroxylamine to additional stabilizer, for example.

The additional stabilizers may include, but are not limited to, dithionites, amines, sulphites, hydroquinones, hydrides, carboxylic acids, piperazines, etc. In one embodiment, the additional stabilizer comprises dithionite salts (e.g., sodium dithionite). In another embodiment, the additional stabilizer comprises ethylenediamine tetraacetic acid (EDTA), methoxy hydroquinone (MEHQ), tetramethyl piperazine-N-oxide (TEMPO), diethylene triamine (DETA), benzaldehyde, sodium sulphite, boric acid, tetraethylene triamine (TETA), sodium borohydride, butylated hydroxyanisole, sodium metabisulfite, ascorbic acid, thiourea, and mixtures thereof.

For example, the stabilizer may comprise dialkyl hydroxylamine, such as aqueous N,N-dialkyl hydroxylamines, as the primary stabilizer, with lesser amounts of additional stabilizer, such as sodium dithionite. For example, from 100 ppm (0.01% wt/wt) to 2000 ppm (0.2% wt/wt) of N,N-diethyl hydroxylamine or a molar equivalent amount of an alternative N,N-diethyl hydroxylamine and about 100 ppm to 2000 ppm of dithionite salt (e.g., sodium dithionite) may be added to an aqueous solution of choline base for the purpose of preventing colour formation and preserving the overall quality of the product. The use of from about 50 ppm (0.005% wt/wt) to 1 g/l or 1000 ppm (0.1% wt/wt) of DEHA combined with 50 ppm to 1 g/l or 1000 ppm of sodium dithionite may be particularly suitable. The use of equal molar amounts of alternative dithionite salts and/or equal molar amounts of another aqueous soluble N,N-dialkyl hydroxylamine may be substituted for sodium dithionite and/or DEHA as would be evident to one of ordinary skill in the art.

Without wishing to be bound to theory, the addition of dialkyl hydroxylamine alone and/or dialkyl hydroxylamine plus an additional stabilizer, such as dithionite, has been found to minimize or eliminate the degradation reactions, which lead to colour formation occurring during the preparation of and storage thereafter of choline hydroxide. Thus, the dialkyl hydroxylamine stabilizer alone or in combination with an additional stabilizer may minimize, slow, or eliminate the Hofmann elimination, oxidation, and/or autoxidation reactions.

### Stabilized Choline Hydroxide Solutions

As described herein, the choline hydroxide solution is stabilized with a sole stabilizer or a combination of stabilizers to provide for minimal or no development of heavy/dark colour. Additionally, the formation of precipitates is also reduced or eliminated. It was discovered that a major influence on choline base stability may be exposure to air (e.g. oxygen) during storage. Without wishing to be bound to a particular theory, oxygen may enhance the rate of Hofmann elimination and/or it may drive a parallel oxidative degradation pathway. Thus, stable, clear colour and reduced precipitation may be due, at least in part, to minimization of the Hofmann elimination reaction in addition to minimization of oxidation, autoxidation, and/or other degradation reactions.

The concentration of choline hydroxide in the solutions may influence the amount of degradation (e.g., colour formation). For example, choline hydroxide solutions having a low concentration of choline hydroxide (e.g. on the order of about 10-15% choline hydroxide) may hardly develop any colour over time (e.g. for weeks or even months). On the other hand, solutions comprising a high concentration of choline hydroxide (e.g. about 45% choline hydroxide in the solution) can develop dark colour very quickly (e.g. on the order of about one day). Thus, the stabilizers described herein may be effective at both low and high concentrations of choline hydroxide. For example, the stabilizers may be effective for solutions containing concentrations of choline hydroxide at 45% choline hydroxide or greater, 40% choline hydroxide or greater, 25% choline hydroxide or greater, 10% choline hydroxide or greater, etc., based on the total amount of stabilized aqueous choline hydroxide solution. In one embodiment, the solution comprises 40-50% by weight choline hydroxide, based on the total amount of stabilized choline hydroxide solution.

The stabilized choline solutions described herein may maintain a clear or slightly off-colour (e.g. APHA of less than 500) appearance for an extended period of time. The colour of the stabilized choline solution may be evaluated by measuring the American Public Health Association (APHA) colour, for example, following appropriate American Society for Testing and Materials (ASTM) procedures (see e.g. ASTM D1209). APHA measurements, expressed herein, were taken using a calibrated Lovibond PFX195 Tintometer with a 5 cm path length quartz cell. The APHA colour value represents a scale ranging from a low, transparent/light to a high, opaque/dark sample. A value less than 20 is indicative of a clear or water-white sample. A value less than 100 is indicative of a clear or slightly off-colour appearance sample. A value less than 500 is indicative of a clear to amber sample. A value greater than 500 is indicative of amber to an opaque dark colour. Thus, a lower value establishes a more clear/lighter sample whereas a higher value designates a more opaque/darker sample. As the darkness and opaqueness represents the presence of degradation reactions and associated byproducts of the choline base, a lower value is desired. In one embodiment of the invention, the stabilized choline hydroxide solution has an APHA colour value of 500 or less, 300 or less, 100 or less, 50 or less, or 20 or less when stored and measured at room temperature.

The stabilized choline solution also has a suitable shelf life. In other words, the choline solution remains stable for an extended period of time, for example, on the order of at least one week, at least one month, at least three months, at least six months, at least one year, at room temperature (e.g., about 20-25°C) and under standard conditions. In an exemplary embodiment, the choline solution remains stable and has an APHA of 100 or less for up to 6 months. Additionally, the stabilized choline solution may also maintain a clear or slightly off-colour (e.g., APHA of less than 500) appearance even at elevated temperatures (e.g., up to 60°C) for limited durations.

In one embodiment of the invention, the stabilized choline hydroxide solution has an APHA colour value of 300 or less (e.g., 100 or less) at room temperature for a duration of at least 6 months after manufacture of the stabilized choline hydroxide solution.

### Methods of Stabilization

According to the invention, a method for the stabilization of an aqueous choline hydroxide solution is defined in claim 1.

For example, an aqueous choline hydroxide solution may be stabilized by first adding a first stabilizer comprising a dithionite salt or a dialkyl hydroxylamine to an aqueous solution containing reactants that will produce an aqueous choline hydroxide solution. In other words, the first stabilizer may be added at any time during the formation of the choline hydroxide. This may include an addition of the stabilizer when the reactants (e.g. trimethylamine (TMA) with ethylene oxide (EO)) are added to the reactor. Additionally, the first stabilizer may be added, for example, incrementally while the reaction is taking place to produce the choline hydroxide solution. Thus, some portion of the stabilizer (i.e. the first stabilizer) is added to the reactants or reaction mixture before or during the reaction to produce the choline solution. The stabilizer(s) may be added at any point when most convenient.

An amount of the first stabilizer from 50 to 500 ppm is added prior to complete formation of the choline hydroxide solution. For example, about 200 ppm to about 500 ppm of the first stabilizer, by weight of the total solution, may be added to the hydroxide solution.

The reaction may be carried out in any suitable apparatus, such as a batch reactor, a continuous stirred tank reactor (CSTR), or in a plug flow reactor, for example.

In batch mode, the ethylene oxide may be fed, for example, at a controlled rate into an aqueous solution of trimethylamine with adjustment of the addition rate so that the temperature remains below an upper set point.

In CSTR mode, the ethylene oxide and aqueous solution of trimethylamine may be fed into the top of a reactor containing an aqueous solution with an excess of trimethylamine while a continuous stream of product may be taken from the bottom of the reactor with distillation and recycle of the excess trimethylamine from the product stream.

In plug flow mode, the ethylene oxide, trimethylamine, and water may be pumped into a tubular reactor at a rate that creates turbulent flow and sufficient mixing of the reactants but at the same time is slow enough relative to the specific reactor configuration to ensure that the reaction temperature does not exceed an upper set point. Often the temperature may be monitored as a function of the distance along the reactor path for the purpose of controlling the rate of reactant(s) addition.

Second, after the aqueous choline hydroxide solution is formed, a second stabilizer comprising a dialkyl hydroxylamine is added to the aqueous choline hydroxide solution. The second stabilizer may be added at any time after formation of the choline hydroxide solution. For example, the second stabilizer may be added as soon as the reaction is complete or some period of time later (e.g. ten minutes later, one hour later, or one day later).

The first stabilizer may be a single stabilizer or may include additional stabilizer(s) (e.g., ethylene diamine tetraacetic acid (EDTA), methoxy hydroquinone (MEHQ), tetramethyl piperazine-N-oxide (TEMPO), diethylene triamine (DETA), benzaldehyde, sodium sulphite, boric acid, tetraethylene triamine (TETA), sodium borohydride, butylated hydroxyanisole, sodium metabisulphite, ascorbic acid, thiourea, and mixtures thereof). The second stabilizer may be a single stabilizer or may include additional stabilizer(s) as described herein.

An amount of the second stabilizer from 200 to 1000 ppm is added after formation of the choline hydroxide solution. For example, about 200 ppm to about 500 ppm of the second stabilizer, by weight of the total choline solution, may be added to the choline hydroxide solution once formed.

In the invention, a greater amount of the second stabilizer is added relative to the amount of the first stabilizer added. In other words, more second stabilizer is added after formation of the choline solution than before or during manufacture. For example, a ratio of the first stabilizer to the second stabilizer may range from about 1:1 to about 1:10 or about 1:1 to about 1:4 first to second stabilizer. It will be recognized to one of ordinary skill in the art that if the first and second stabilizers are the same, a total amount of the sole stabilizer may be divided as appropriate between pre- and post- additions (e.g. some amount of the sole stabilizer is the "first" stabilizer and some amount of the sole stabilizer is the "second" stabilizer).

By adding a stabilizer or stabilizers consistent with the invention, before and after formation of the aqueous choline hydroxide solution, a stable choline hydroxide solution may be formed with a low APHA colour evidencing little or no degradation of the choline hydroxide solution. Additionally, these stabilized choline hydroxide solutions remain stabilized for durations necessary for a good shelf life.

### EXAMPLES

### Example 1: Preparation of Choline Hydroxide Solution

Water (330 g) and stabilizer (as provided in the tables) were added to a 1L stainless steel batch reactor (pre-addition). In cases where the stabilizer hydrolyzes at neutral/acid pH, the water was pre-treated with some trimethylamine (TMA) in order to increase the pH. The reactor was closed and the gas phase in the reactor was replaced with nitrogen by flushing three times. Gaseous trimethylamine (TMA, 117 g) was bubbled into the reaction mixture and then the total pressure was increased to 10 barg with nitrogen. The mixture was stirred and heated to 30°C. Ethylene oxide (EO, 88 g) was pumped slowly into the reactor at such a rate that the temperature remained below 35°C (concentration of free EO in the gas phase being kept below ≈ 10% for safety reasons). After all the EO was pumped into the reactor, the reaction mixture was stirred for an additional 1 h at 30°C. The reaction mixture was cooled and degassed with nitrogen until the residual TMA level was reduced to below 100 ppm. The resulting solution theoretically contains a concentration of 45% choline hydroxide. The product was, if applicable, treated with an additional portion of stabilizer (post-addition) and then stored under nitrogen in a cool dark place. The colour was evaluated on a periodic basis.

APHA measurements were made by use of a calibrated Lovibond PFX195 Tintometer with a 5 cm path length quartz cell.

### Comparative Example 2: Choline Base Colour Stabilizers and

Table 1 below refers to aqueous choline base solutions prepared as described above. The choline base solutions were blanketed with nitrogen and stored at elevated temperature (60°C). Colour was judged by visual assessment through a 4 cm path length on a scale of 0 through 4 with 0 being clear and water-white (APHA < 20); 1 being clear and slightly off-colour appearance (APHA < 100); 2 being clear and amber (APHA < 500); 3 being almost opaque and dark amber (APHA > 500, but amber colour discernible); 4 being opaque and dark (APHA > 500, sample appears to black). The abbreviations are as follows: DEHA = diethyl hydroxylamine; EDTA = ethylenediamine tetraacetic acid; MEHQ = methoxy hydroquinone; TEMPO = tetramethyl piperazine-N-oxide; and DETA = diethylene triamine.

**Table 1**

| Stabilizer | Amount (ppm) Pre-Addition | Amount (ppm) Post-Addition | Color at Days after start | | | | | |
|---|---|---|---|---|---|---|---|---|
| | | | 0 | 1 | 3 | 4 | 8 | 15 |
| None | - | - | 1 | 4 | 4 | 4 | 4 | 4 |
| Sodium Dithionite | 0 | 1000 | 1 | 1 | 1 | 1 | 1 | 1 |
| | 200 | 0 | 0 | - | 3 | 3 | 3 | 3 |
| | 200 | 800 | 0 | - | 0 | 0 | 0 | |
| DEHA + EDTA | 0 | 500/500 | 1 | 1 | 1 | 2 | 2 | 2 |
| DEHA | 0 | 1000 | 1 | 1 | 1 | 2 | 2 | 2 |
| Benzaldehyde | 0 | 1000 | 1 | 4 | 4 | 4 | 4 | 4 |
| Salicylic Acid | 0 | 1000 | 1 | 3 | 4 | 4 | 4 | 4 |
| MEHQ | 0 | 1000 | 1 | 1 | 1 | 2 | 2 | 2 |
| Piperazine | 0 | 1000 | 1 | 3 | 4 | 4 | 4 | 4 |
| TEMPO | 0 | 1000 | 1 | 3 | 3 | 4 | 4 | 4 |
| Sodium Sulfite | 0 | 1000 | 1 | 1 | 1 | 2 | 2 | 2 |
| DETA | 0 | 1000 | 1 | 1 | 2 | 3 | 4 | 4 |
| Boric Acid | 0 | 1000 | 1 | 4 | 4 | 4 | 4 | 4 |

The use of no stabilizer (none) results in deep colour at very short time intervals. The use of alternative stabilizers is markedly less effective. The stabilizing effect of added dithionite, especially with a pre- and post- addition, is clearly seen even at elevated temperatures (60°C).

### Comparative Example 3: Post-Addition Choline Base Colour Stabilizers

Table 2 below refers to aqueous choline base solutions prepared as described above in Example 1 but without in-process stabilizer added. The stabilizer was added at the end (i.e. only post-addition).

**Table 2**

| Stabilizer | APHA Color | | |
|---|---|---|---|
| | 0 Hrs | 22°C | 60°C |
| | | 16 Hrs | 2 Hrs |
| DEHA (1000 ppm) | 252 | 440 | >500 |
| sodium dithionite (1000 ppm) | 252 | 390 | 440 |
| sodium borohydride/ butylated hydroxyanisole (500 ppm each) | 252 | 485 | >500 |
| sodium metabisulphite (1000 ppm) | 252 | 440 | >500 |
| Ascorbic Acid (1000 ppm) | 252 | >500 | >500 |
| Thiourea (1000 ppm) | 252 | >500 | >500 |
| No Stabilizer | 252 | >500 | >500 |

### Comparative Example 4: Efficacy of Stabilizers at Different Temperatures

Table 3 provides aqueous choline base solutions prepared as described in Example 1 above at room temperature (T = 20°C) and an elevated storage temperature (T = 60°C).

**Table 3**

| Pre-treatment Stabilizer | Post-treatment Stabilizer | Time | APHA Colour |
|---|---|---|---|
| T = 20°C | | | |
| 200 ppm sodium dithionite | 200 ppm sodium dithionite | 3 days | 9 |
| | | 6 days | 6 |
| | | 17 days | 3 |
| 200 ppm sodium dithionite | 500 ppm sodium dithionite | 3 days | 3 |
| | | 6 days | 17 |
| | | 17 days | 6 |
| 200 ppm sodium dithionite | 800 ppm sodium dithionite | 3 days | 4 |
| | | 6 days | 11 |
| | | 17 days | 3 |

| T = 60°C | | | |
|---|---|---|---|
| 200 ppm sodium dithionite | 200 ppm sodium dithionite | 3 days | 5 |
| | | 6 days | 15 |
| | | 8 days | 76 |
| | | 13 days | 288 |
| | | 17 days | 291 |
| 200 ppm sodium dithionite | 500 ppm sodium dithionite | 3 days | 3 |
| | | 6 days | 9 |
| | | 8 days | 32 |
| | | 13 days | 188 |
| | | 17 days | 233 |
| 200 ppm sodium dithionite | 800 ppm sodium dithionite | 3 days | 5 |
| | | 6 days | 3 |
| | | 8 days | 21 |
| | | 13 days | 143 |
| | | 17 days | 182 |

The stabilizing effect of dithionite with pre- and post- additions is clearly seen.

### Example 5: Efficacy of Different Stabilizers Pre- and/or Post-Treatment

Table 4 provides aqueous choline base solutions prepared as described in Example 1 above with different and varying amounts of pre- and post- stabilizers.

**Table 4**

| Pre-treatment Stabilizer | Post-treatment Stabilizer | Time | Visual Colour |
|---|---|---|---|
| none | none | Start | Very Dark |
| | | 1 day | Very Dark |
| | | 7 days | Very Dark |
| | | 41 days | Very Dark |
| 200 ppm sodium dithionite | none | Start | Clear |
| | | 1 day | Clear |
| | | 7 days | Clear |
| | | 41 days | Clear |
| 200 ppm sodium dithionite | 300 ppm sodium dithionite | Start | Clear |
| | | 1 day | Clear |
| | | 7 days | Clear |
| | | 41 days | Clear |
| 200 ppm sodium dithionite | 300 ppm sodium dithionite & 500 ppm TETA | Start | Clear |
| | | 1 day | Clear |
| | | 7 days | Clear |
| | | 41 days | Clear |
| 200 ppm sodium dithionite | 800 ppm TETA | Start | Clear |
| | | 1 day | Clear |
| | | 7 days | Clear |
| | | 41 days | Clear |
| 200 ppm DEHA | none | Start | Clear |
| | | 1 day | Clear |
| | | 7 days | Amber |
| | | 41 days | Dark |
| 200 ppm DEHA | 300 ppm DEHA | Start | Clear |
| | | 1 day | Clear |
| | | 7 days | Clear |
| | | 41 days | Light Amber |
| 200 ppm DEHA | 800 ppm DEHA | Start | Clear |
| | | 1 day | Clear |
| | | 7 days | Clear |
| | | 41 days | Clear |
| 200 ppm DEHA | 300 ppm DEHA & 500 ppm TETA | Start | Clear |
| | | 1 day | Clear |
| | | 7 days | Clear |
| | | 41 days | Clear |
| 200 ppm DEHA | 800 ppm TETA | Start | Clear |
| | | 1 day | Clear |
| | | 7 days | Clear |
| | | 41 days | Clear |
| 200 ppm DEHA | 800 ppm Sodium Dithionite | Start | Clear |
| | | 1 day | Clear |
| | | 7 days | Clear |
| | | 41 days | Clear |

## Claims

1. A method for the stabilization of an aqueous choline hydroxide solution comprising:
- adding a first stabilizer comprising a dialkyl hydroxylamine to a previous aqueous solution containing reactants that eventually will produce the aqueous choline hydroxide solution in an amount from 50 ppm to 500 ppm by weight of said previous aqueous solution; and
- after the aqueous choline hydroxide solution has been formed, adding a second stabilizer comprising a dialkyl hydroxylamine to the aqueous choline hydroxide solution in an amount from 200 ppm to 1000 ppm by weight of the aqueous choline hydroxide solution and wherein a greater amount of the second stabilizer is added relative to the amount of the first stabilizer added.

2. The method according to claim 1 wherein the second stabiliser comprises N,N-diethyl hydroxylamine.

3. The method according to claim 1 or claim 2, wherein the first and/or the second stabilizer further comprises a dithionite salt.

4. The method according to any one of the preceding claims, wherein a weight ratio of the first stabilizer to the second stabilizer ranges from 1:1 to about 1:10.

5. The method according to any one of the preceding claims, wherein the first stabilizer or the second stabilizer comprises an additional stabilizer selected from the group consisting of ethylene diamine tetraacetic acid (EDTA), methoxy hydroquinone (MEHQ), tetramethyl piperazine-N-oxide (TEMPO), diethylene triamine (DETA), benzaldehyde, sodium sulphite, boric acid, tetraethylene triamine (TETA), sodium borohydride, butylated hydroxyanisole, sodium metabisulphite, ascorbic acid, thiourea, and mixtures thereof.

6. The method according to any one of the preceding claims, wherein the first stabilizer or the second stabilizer consists of N,N-diethyl hydroxylamine.

7. A stabilized choline hydroxide solution obtained by a method according to any one of the claims 1 to 6.

8. The stabilized choline hydroxide solution according to claim 7, wherein the stabilized choline hydroxide solution comprises 40-50% by weight choline hydroxide.

## Patentansprüche

1. Ein Verfahren zur Stabilisierung einer wässrigen Cholinhydroxidlösung, welches Folgendes umfasst:
- Hinzufügen eines ersten Stabilisators, der Dialkylhydroxylamin umfasst, zu einer vorigen wässrigen Lösung, welche Reaktanten enthält, die letztendlich die wässrige Cholinhydroxidlösung in einer Menge von 50 bis 500 Gew.-ppm der erwähnten vorigen wässrigen Lösung produzieren werden; und
- nachdem die wässrige Cholinhydroxidlösung gebildet wurde, Hinzufügen eines zweiten Stabilisators, der Dialkylhydroxylamin umfasst, zur wässrigen Cholinhydroxidlösung in einer Menge von 200 bis 1.000 Gew.-ppm der wässrigen Cholinhydroxidlösung und wobei eine größere Menge des zweiten Stabilisators im Verhältnis zur Menge des ersten hinzugefügten Stabilisators hinzugefügt wird.

2. Das Verfahren nach Anspruch 1, wobei der zweite Stabilisator N,N-Diethylhydroxylamin umfasst.

3. Das Verfahren nach Anspruch 1 oder Anspruch 2, wobei der erste und/oder der zweite Stabilisator ferner ein Dithionitsalz umfasst.

4. Das Verfahren nach irgendeinem der vorigen Ansprüche, wobei ein Gewichtsverhältnis des ersten Stabilisators zum zweiten Stabilisator von 1:1 bis ungefähr 1:10 reicht.

5. Das Verfahren nach irgendeinem der vorigen Ansprüche, wobei der erste Stabilisator oder der zweite Stabilisator einen zusätzlichen Stabilisator umfasst, ausgewählt aus der Gruppe bestehend aus Ethylendiamintetraessigsäure (EDTA), Methoxyhydrochinon (MEHQ), Tetramethylpiperazin-N-oxid (TEMPO), Diethylentriamin (DETA), Benzaldehyd, Natriumsulfit, Borsäure, tetraethylentriamin (TETA), Natriumborhydrid, Butylhydroxyanisol, Natriummetabisulfit, Ascorbinsäure, Thioharnstoff, und Mischungen davon.

6. Das Verfahren nach irgendeinem der vorigen Ansprüche, wobei der erste Stabilisator oder der zweite Stabilisator aus N,N-Diethylhydroxylamin besteht.

7. Eine stabilisierte Cholinhydroxidlösung, erhalten durch ein Verfahren nach irgendeinem der Ansprüche 1 bis 6.

8. Die stabilisierte Cholinhydroxidlösung nach Anspruch 7, wobei die stabilisierte Cholinhydroxidlösung 40 bis 50 Gew.-% Cholinhydroxid umfasst.

## Revendications

1. Procédé de stabilisation d'une solution aqueuse d'hydroxyde de choline comprenant les étapes consistant à :
- ajouter un premier stabilisant comprenant une dialkylhydroxylamine à une solution aqueuse précédente contenant des réactifs qui produiront finalement la solution aqueuse d'hydroxyde de choline en une quantité de 50 ppm à 500 ppm en poids de ladite solution aqueuse précédente ; et
- après que la solution aqueuse d'hydroxyde de choline a été formée, ajouter un second stabilisant comprenant une dialkylhydroxylamine à la solution aqueuse d'hydroxyde de choline en une quantité de 200 ppm à 1000 ppm en poids de la solution aqueuse d'hydroxyde de choline et où une quantité supérieure du second stabilisant est ajoutée relativement à la quantité du premier stabilisant ajouté.

2. Procédé selon la revendication 1, dans lequel le second stabilisant comprend de la N,N-diéthylhydroxylamine.

3. Procédé selon la revendication 1 ou la revendication 2, dans lequel le premier et/ou le second stabilisant comprend en outre un sel de dithionite.

4. Procédé selon l'une quelconque des revendications précédentes, dans lequel un rapport de poids du premier stabilisant au second stabilisant est compris entre 1 : 1 et environ 1:10.

5. Procédé selon l'une quelconque des revendications précédentes, dans lequel le premier stabilisant ou le second stabilisant comprend un stabilisant supplémentaire choisi dans le groupe constitué de : éthylènediaminetétraacétique (EDTA), méthoxyhydroquinone (MEHQ), N-oxyde de tétraméthylpipérazine (TEMPO), diéthylènetriamine (DETA), benzaldéhyde, sulfite de sodium, acide borique, tétraéthylènetriamine (TETA), borohydrure de sodium, hydroxyanisol butylé, métabisulfite de sodium, acide ascorbique, thiourée et leurs mélanges.

6. Procédé selon l'une quelconque des revendications précédentes, dans lequel le premier stabilisant ou le second stabilisant est constitué de N,N-diéthylhydroxylamine.

7. Solution stabilisée d'hydroxyde de choline obtenue par un procédé selon l'une quelconque des revendications 1 à 6.

8. Solution stabilisée d'hydroxyde de choline selon la revendication 7, dans laquelle la solution stabilisée d'hydroxyde de choline comprend de 40 à 50 % en poids d'hydroxyde de choline.
